# EUROPÄISCHE PATENTSCHRIFT

(11) **EP 0 367 002 B1**
(45) Veröffentlichungstag und Bekanntmachung des Hinweises auf die Patenterteilung: **15.06.1994**
(21) Anmeldenummer: 89119137.1
(22) Anmeldetag: 14.10.1989
(51) Int. Cl.: C08B 5/00, C08B 31/02, C08B 13/00, C12N 11/12, B01J 20/22

(54) **Kohlensäureester von Polysacchariden und Verfahren zu ihrer Herstellung**
Carbonic-acid esters of polysaccharides and process for their preparation
Esters d'acide carbonique de polysaccharides et procédé pour leur préparation

(30) Priorität: 27.10.1988 DE 3836600
(43) Veröffentlichungstag der Anmeldung: 09.05.1990
(73) Patentinhaber: Wolff Walsrode Aktiengesellschaft, D-29655 Walsrode (DE)
(72) Erfinder: Buysch, Hans-Josef, Dr., D-4150 Krefeld (DE); Klausener, Alexander, Dr., D-4150 Krefeld (DE); Szablikowski, Klaus, Dr., D-3030 Walsrode (DE); Balser, Klaus, Dr., D-3030 Walsrode (DE); Wilke, Michaela, Dr., D-3043 Schneverdingen (DE)
(74) Vertreter: Zobel, Manfred, Dr.

(56) Entgegenhaltungen:
- FR-A- 2 073 901
- FR-A- 2 335 525
- US-A- 3 284 442

## Beschreibung

Die vorliegende Erfindung betrifft neue Kohlensäureester von Polysacchariden mit einem Substitutionsgrad (DS) von 0,5 und größer und Verfahren zu ihrer Herstellung.

Kohlensäureester von Polysacchariden sind interessante Ausgangsprodukte zur Herstellung weiterer modifizierter Polysaccharide, besonders von Carbamaten mit unterschiedlichen Eigenschaften. Eine besonders aktuelle Verwendung finden sie zur Herstellung von Depots für bioaktive Materialien (vgl. Makromol. Chem. 186, 17-29 (1985)) und zur Fixierung von Enzymen für die Durchführung enzymatischer Reaktionen in heterogener Phase (vgl. z.B. J.C.S. Perkin I 1974, 757-762 oder Biochemistry Internat. 4 (1982) 629-635). Darum hat es nicht an Bemühungen gefehlt, Kohlensäureester verschiedener Polysaccharide herzustellen. Für die genannten Anwendungen, besonders die Fixierung von Enzymen ist ein hoher DS wünschenswert, damit eine hohe Enzymdichte am Polysaccharid, hohe Aktivität und geringe Katalysatorvolumina für die enzymatischen Reaktionen gewährleistet sind.

Der überwiegende Teil der bisherigen Verfahren arbeitet in homogener Phase, also mit gelöstem Polysaccharid, wobei in der Regel nur niedrig substituierte Polysaccharide erhalten werden. Darüber hinaus bringt die Umsetzung in homogener Phase wegen der häufig sehr hohen Viskosität solcher Lösungen und der daher notwendigen Verwendung riesiger Mengen an Lösungsmitteln bei der Reaktion und Ausfällung der umgesetzten Polymeren große Probleme mit sich.

Nach E. Heuser und F. Schneider, Ber. deut. chem. Ges. 57, 1389-1392 (1924) soll man durch homogene Reaktion von aus Viskose gewonnener Hydratcellulose in 8 %iger Natronlauge eine Methylcarbonatcellulose mit einem DS von maximal 2 erhalten, der aber wegen der Anwesenheit der verseifenden Natronlauge ein Zufallsergebnis und nicht reproduzierbar ist. Die Ausbeuten liegen immer unter 20 %. Damit ist dieses Verfahren als völlig unbrauchbar einzustufen.

In Lösungen von Dimethylsulfoxid lassen sich Stärke, Dextrin und Dextran (Carbohydrate Research 8 (1968), 266-274) bis zu einem maximalen DS von 0,4 in Carbonat-Polysaccharide überführen. Heterogene Umsetzungen von Cellulose in Dimethylsulfoxid oder Dimethylformamid führen nach Carbohydrate Research 17 (1971), 471-4 aufgrund spektroskopischer Analysen zu einem DS um 0,5. Der nach Umsetzung des Carbonats mit NH₃ zum Carbamat zu ermittelnde Stickstoffwert von 0,8 läßt allerdings einen wesentlich geringeren DS von <0,2 vermuten.

In J.C.S. Perkin I 1973, 2293-2299 wird berichtet, daß makroporöse Cellulose ebenfalls in einer Suspension in Dimethylsulfoxid nach Umsetzung mit Chlorkohlensäureethylester zu einem Cellulosecarbonat von DS <0,5 führt (errechnet aus der maximalen NH₃-Aufnahme 3 mmol/g Matrix).

Dextrane lassen sich in einer Lösung von Dimethylformamid, die LiCl enthält, mit Chlorkohlensäureethyl- oder -butylester in Gegenwart verschiedener Basen hoch substituieren bis zu einem DS von 3,0, s. Makromol. Chemie (1985) 17-29, der allerdings aus spektroskopischen Daten errechnet wurde. Die Angaben des Carbonatgehaltes in Mol-% lassen allerdings auf wesentlich geringere Substitutionsgrade im Bereich von maximal 1,5 schließen. In dieser Publikation wird darauf hingewiesen, daß die Reaktionsgeschwindigkeit und der Substitutionsgrad von der Basizität der verwendeten Hilfsbase abhängt. Demnach werden die besten Ergebnisse mit Triethylamin erhalten. Trotz der hohen Substitutionsgrade ist dieses Verfahren aus den o.g. Gründen wegen des komplexen Lösungsmittelgemisches und der damit verbundenen schwierigen Aufarbeitung für technische Zwecke ungeeignet. Außerdem erfolgt unter den Reaktionsbedingungen eine Zersetzung der Acylhalogenide.

Verwendet man aromatische Chlorkohlensäureester, z.B. den p-Nitrophenylester, und arbeitet in Dimethylsulfoxid als Lösungsmittel für Dextran, so findet man einen wesentlich geringeren DS von ca. 0,3, wie sich aus der prozentualen Aktivierung von max. 30 % (Anzahl der Carbonatgruppen pro 100 Anhydroglukoseeinheiten) errechnet. Außerdem zeigt sich, daß die Anzahl der Carbonatgruppen während der Reaktion durch ein Maximum verläuft, also die Carbonatgruppe unter den Reaktionsbedingungen nicht beständig ist (Makromol. Chem. 186, 2455 bis 2460 (1985)).

Ähnliches gilt für Umsetzungen mit aromatischen Chlorkohlensäureestern an gelförmiger Sepharose, die in trockenem Pyridin/Aceton-Gemisch suspendiert wird (Biochemistry International 4 (1982), 629-635). Man erhält nur einen geringen DS bis zu 0,28 (errechnet aus der Angabe von max. 1,8 mmol aktiven Gruppen pro g Polymer).

Aus FR-A 2 335 525 und FR-A 2 073 901 sind Verfahren zur Hersteilung von Celluloseethern durch Umsetzung von Celluloseethern mit Chlorkohlensäureestern bekannt. Die Verfahren sind jedoch nicht geeignet, Derivate mit höheren Substitutionsgraden herzustellen. Darüber hinaus werden nach dem Verfahren der FR-A 2 073 901 Substanzen erhalten, die einen sehr hohen Anteil an cyclischen Carbonat-Gruppierungen enthalten.

Nach dem derzeitigen Stand ist kein Verfahren verfügbar, nach dem man in heterogener Phase, d.h. bei relativ hoher Konzentration an Polysaccharid unter Vermeidung von hochviskosen Lösungen hochsubstituierte Polysaccharid-Carbonate herstellen könnte, insbesondere fehlen Verfahren zur Herstellung von hochsubstituierten, aromatischen Polysaccharid-Carbonaten, die verschieden reaktive Carbonatbindungen aufweisen und für weitere Substitutionsreaktionen besonders geeignet sind. Nichtdestoweniger besteht ein hohes technisches Interesse an derartigen Polymeren (Carbohydrate Research 26 (1973), 401 bis 408, S. 402 und 407).

Aufgabe der Erfindung war es, neue hochsubstituierte Polysaccharidcarbonate bereitzustellen.

Gegenstand der Erfindung sind daher Polysaccharidcarbonate mit wiederkehrenden Einheiten der Formel I 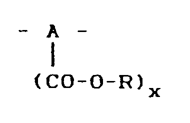
worin bedeuten
- A: eine Cellulose-, Methylcellulose-, Ethylcellulose-, Hydroxyethylcellulose-, Hydroxypropylcellulose-, Carboxymethylcellulose-, Na-, K-, Ca-, NH₄-, quartäre Ammoniumsalze der Carboxymethylcellulose-, Na-, K-, Ca-, NH₄-, quart. Ammonium-Cellulosesulfat-, Stärke-, Dextrin-, Glycogen-, Fructose-, Inulin-, Graminin-, Mannose-, Galactosan-, Hemicellulose-, Xylose-, Arabinose-, Gelfan-, Xanthan- oder Pullulan-Einheit
- R: einen aliphatischen oder cycloaliphatischen Rest mit 1 bis 18 C-Atomen, einen araliphatischen Rest mit 7 bis 12 C-Atomen, einen gegebenenfalls mit Halogen, NO₂, Phenyl COOR¹, OR¹ oder einen mit einem C₁-C₆-Aliphaten substituierten aromatischen Rest mit 6 bis 12 C-Atomen
- R¹: Alkyl mit 1 bis 4 C-Atomen
- x: eine Zahl von 0,5 bis 3,0
wobei die Cellulose- bzw. Cellulosederivat- bzw. Stärkecarbonate bei 20°C zu maximal 20 Gew.-% in Chloroform löslich sind und von den Carbonatgruppen maximal 30 % cyclische Carbonatgruppen sind.

In einer bevorzugten Ausführungsform bedeuten:
- R: einen aliphatischen Rest mit 1 bis 6 C-Atomen, einen aromatischen Rest mit 6 bis 10 C-Atomen, gegebenenfalls substituiert mit Cl, CH₃, NO₂, OCH₃ und COOCH₃ und
- x: eine Zahl von 0,8 bis 3,0,
- A: eine Monosaccharideinheit abgeleitet von einer Hexose oder Pentose.

In einer besonders bevorzugten Ausführungsform bedeuten:
- R: CH₃, C₂H₅, Phenyl, Chlorphenyl,Nitrophenyl und Naphthyl und
- x: eine Zahl von 1,0 bis 3,0,
insbesondere
- R: Phenyl und
- x: eine Zahl von 1,0 bis 3,0.

Gegenstand der vorliegenden Erfindung ist auch ein Verfahren zur Herstellung von Polysaccharidcarbonaten durch Umsetzung von Polysacchariden mit Kohlensäureestern, vorzugsweise Chlorkohlensäureester, in Gegenwart einer Base, dadurch gekennzeichnet, daß man das Polysaccharid in heterogener Phase gegebenenfalls nach Vorbehandlung mit der Base in Gegenwart von vorzugsweise mindestens 0,5 Gew.-% Feuchtegehalt, bezogen auf das Polysaccharid, mit Kohlensäureester bei Temperaturen zwischen vorzugsweise 10 und 120°C vorzugsweise mindestens 3 Stunden reagieren läßt.

Gegenstand der Erfindung sind auch Polysaccharidcarbonate hergestellt durch Acylierung von Polysacchariden mit Kohlensäureestern in Gegenwart einer Base in heterogener Phase, dadurch gekennzeichnet, daß die Polysaccharidcarbonate einen Substitutionsgrad von 0,5-3,0 bezogen auf die Monosaccharideinheit aufweisen, die Polysaccharidcarbonate bei 20°C zu maximal 20 Gew.-% in Chloroform löslich sind und das von den Carbonatgruppen maximal 30 % bevorzugt maximal 20 % besonders bevorzugt maximal 10 % cyclisch sind.

Nach dem Stand der Technik muß dies in mehr als einer Hinsicht überraschen: Zunächst sind selbst in homogener Lösung von Polysacchariden nur in Ausnahmefällen hohe Carbonatgehalte zu erreichen. Die bisher offensichtlich besonders schwierig zugänglichen aromatischen Carbonate lassen sich trotz der größeren Raumerfüllung der aromatischen Reste sehr gut herstellen. Nach den übereinstimmenden Verfahrensweisen (niedrige Temperatur, kurze Reaktionszeit) aller bisherigen Bearbeiter und nach den vorliegenden Beobachtungen der Zersetzlichkeit der Carbonate und der Chlorkohlensäureester wären unter den erfindungsgemäßen Bedingungen kaum Carbonatsubstitution, schon gar nicht hohe Substitutionsgrade zu erwarten gewesen. Erstaunlich ist auch, daß keine cyclischen Carbonate gebildet werden, was nach vorliegenden Ergebnissen offensichtlich schwer zu vermeiden ist. Besonders aromatische Carbonate sollten wegen der lockeren Bindung des aromatischen Teils leicht in cyclische Carbonate übergehen.

Geeignete Ausgangsmaterialien zur Herstellung der erfindungsgemäßen Polysaccharidcarbonate sind beispielsweise Cellulose, Methylcellulose oder gemischte Celluloseether wie Methyl-hydroxyethyl-cellulosen, Carboxymethylcellulose, ihre verschiedenen Salze mit Natrium-, Kalium-, Calcium- oder Ammonium- besonders quartären Ammoniumionen; Cellulosesulfat mit verschiedenen Gegenionen, etwa des Natriums, Kaliums, Calciums, Ammoniums und quartärer Ammoniumgruppen;
Stärke, Dextrin, Glycogen;
Fructosan, Inulin und Graminin;
Mannosan, Galactosan;
Hemicellulosen ferner Xylosan und Arabinosan, sowie auch Heteropolysaccharide wie Gellan, Xanthan und Pullulan.

Bevorzugte Ausgangsprodukte sind Cellulose, Stärke, und Dextrin, besonders bevorzugt sind Cellulose, Methylcellulose, Ethylcellulose, Hydroxyethylcellulose, Hydroxypropylcellulose, Carboxymethylcellulose und deren Salze und Stärke.

Geeignete Kohlensäureester sind insbesondere solche der allgemeinen Formel 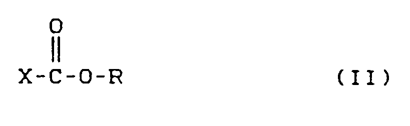
worin bedeuten
- R: wie bei der Formel (I) angegeben
- X: Brom, Azol, insbesondere Triazol, Imidazol, Pyridinium, insbesondere Chlor.

Geeignete Basen für die Herstellung der erfindungsgemäßen Polysaccharid-Carbonate sind tertiäre Stickstoffbasen aus der aliphatischen, aromatischen und heterocyclischen Reihe wie Trimethylamin, Triethylamin, Tributylamin, Dimethyl-cyclohexylamin, Diisopropyl-ethylamin, Dicyclohexyl-methylamin, Dimethyl-β-methoxyethylamin, N,N,N′,N′-Tetramethylethylendiamin, N,N,N′,N′-Tetramethyl-β,β′-diamino-diethylether, N,N′-Dimethylpiperazin, N-Methyl-morpholin und Diazabicyclooctan, N,N-Dimethylanilin, N,N-Diethylanilin, N,N,N′,N′-Tetramethyldiaminobenzol, N,N-Dimethyl-toluidin, N,N-Diethylxylidin, N,N-Dimethylanisidin und N-Phenylmorpholin, Pyrazol, N-Alkylpyrazol, Imidazol, N-Methylimidazol, Triazol, N-Ethyltriazol, N,N-Dimethylamino-imidazol, N,N-Diethylamino-triazol, Pyridin, α, β-, γ-Picolin, die Lutidine, Collidin, Ethyl-methylpyridine, N,N-Dialkylaminopyridine wie N,N-Dimethyl-4-aminopyridin, Chinolin, Methylchinolin und Isochinolin.

Bevorzugt sind Triethylamin, Dimethylcyclohexylamin, Methylmorpholin, Dimethylpiperazin und Diazabicyclooctan, Tetramethylethylendiamin, N,N-Dimethylanilin und N,N-Dimethylanisidin, Imidazol, N-Methylimidazol, N,N-Dimethylamino-imidazol, Pyridin, Chinolin und die Methyl- und Ethylpyridine, besonders bevorzugt aber sind Pyridin und die Methylpyridine.

Das Molverhältnis von Kohlensäureester zu Monosaccharideinheit ist variabel und hängt davon ab, welcher Substitutionsgrad erreicht werden soll. Im allgemeinen sollte das Molverhältnis mindestens 0,8:1 betragen. Entsprechend sollte zur Erzielung höherer Substitutionsgrade ein Überschuß an Kohlensäureester pro Mol Saccharid-Einheit eingesetzt werden. Das Molverhältnis kann bis zu 10:1 betragen. Häufig ist dies jedoch wenig ökonomisch, so daß Verhältnisse zwischen 0,8:1 bis 6:1, bevorzugt 1:1 bis 4:1, eingesetzt werden.

Selbstverständlich kann man nach dem erfindungsgemäßen Verfahren auch Substitutionsgrade unter 0,5 einstellen. Dazu müssen dann entsprechend geringere Mengen an Kohlensäureester verwendet oder die Reaktionszeiten verkürzt werden.

Das Molverhältnis von Base zu Kohlensäureester sollte mindestens 1:1 betragen. Man kann auch einen Überschuß an Base einsetzen, so daß das Molverhältnis von Base zu Kohlensäureester zwischen etwa 1:1 bis 10:1 liegen kann. So ist es möglich, die Reaktion ohne zusätzliches Dispersionsmedium in einem Überschuß der Base vorzunehmen.

Die Reaktion kann also mit und ohne zusätzliches Dispersionsmedium durchgeführt werden. Geeignete Dispersionsmedien sind unter den Reaktionsbedingungen inerte Lösungsmittel wie aliphatische und aromatische Kohlenwasserstoffe, Halogenkohlenwasserstoffe, Ether, Ester, Ketone, Carbonsäureamide und Carbonsäurenitrile.

Genannt seien beispielsweise Cyclohexan, Pentan, Heptan, Isooctan, Benzol, Toluol, Methylenchlorid, Chloroform, Dichlorethan, Trichlorethylen, Chlorbenzol, Dichlorbenzol, Brombenzol, Diethylether, Diisopropylether, Dibutylether, Dioxan, Benzodioxan, Anisol, Dimethoxybenzol, Ethylenglykoldimethylether, Diethylenglykol-dimethylether, Essigsäureethylester, Essigsäurebutylester, Propionsäureethylester, Buttersäureethylester, Benzoesäureethylester, Malonsäurediethylester, Bernsteinsäurediethylester, Aceton, Methylethylketon, Diethylketon, Methylisopropylketon, Acetophenon, Cyclohexanon, Formamid, Methylformamid, Dimethylformamid, Dimethylacetamid, Tetramethylharnstoff, N,N-Dimethyl-ethylenharnstoff, N,N-Dimethyl-2,6-diaza-4-oxa-cyclohexanon, N-Methylpyrrolidon, N-Methylcaprolactam, Acetonitril, Propionitril, Butyronitril, Benzonitril, Adiponitril, β-Methoxy-propionitril und β-Cyano-β′-methoxydiethylether.

Die Dispersionsmediummenge soll so bemessen sein, daß sich eine gut rührbare Suspension bildet.

Ausgangsmaterialien, Reagentien, Basen und Solventien sollten im wesentlichen wasserfrei sein. Deshalb ist es erforderlich, die Reaktionspartner und Dispersionsmedien von überschüssigem Wasser zu befreien oder eine entsprechend größere Menge an Chlorkohlensäureester zu verwenden, um damit überschüssiges Wasser zu kompensieren. Dies ist jedoch ein wenig ökonomisches Verfahren. Vorteilhafter ist eine Trocknung der gesamten Einsatzmaterialien mit üblichen Mitteln und Methoden, beispielsweise bei erhöhter Temperatur im Vakuum, durch azeotrope Destillation, durch Trocknen über Alkalihydroxiden wie NaOH oder KOH oder Erdalkalihydroxiden oder -oxiden, wie Ca(OH)₂ oder CaO, über entwässerten Salzen wie Natriumsulfat oder Kupfersulfat oder über Molekularsieben, die sich leicht regenerieren lassen.

Wird dabei das Wasser soweit entzogen, daß die Polysaccharide nur noch das nicht entfernbare gebundene Wasser enthalten, so ist die Reproduzierbarkeit der Umsetzung gefährdet und man setzt deshalb mindestens 0,5 Gew.-% bezogen auf das umzusetzende Polysaccharid zu. Der Wasserzusatz ist nach oben durch ökonomische Überlegungen begrenzt, da ein Teil des Acylierungsmittels verloren geht, deshalb gibt man in der Regel 0,5 bis 5 Gew.-%, bevorzugt 0,8 bis 4,0 Gew.-%, besonders bevorzugt 1,0 bis 3,0, hinzu. Der Kristallwasser-Gehalt von Polysacchariden ist unterschiedlich und kann durch geeignete Analysenmethoden bstimmt werden (vgl. "Starch Chemistry and Technology" ed. by R.L. Whistler, J.N. Bemiller, E.F. Paschall 1983 sec. Ed. S. 214 bis 219, Academic Press Inc. N.Y.).

Der Feuchtegehalt von Cellulose wird nach der Analysenmethode vom Verein der Zellstoff- und Papier-Chemiker und -Ingenieure, Merkblatt IV/42/67 vom 23.10.1967 bestimmt.

Für Cellulose beträgt er beispielsweise je nach Typ und Herkommen zwischen 2 und 4 Gew.-%. Angaben über gebundenes Wasser oder Feuchtegehalt können der einschlägigen Literatur entnommen werden (vgl. K. Götze: Chemiefasern, Bd. 1, S. 264, 3. Aufl. 1964 Springer Verlag).

Da der Ester in der Regel wasserfrei ist, wird die fehlende Wassermenge im allgemeinen der Polysaccharidsuspension vor der Dosierung des Esters beigemischt. Die Reaktion wird zweckmäßigerweise so durchgeführt, daß man das Polysaccharid mit der Base, gegebenenfalls zusätzlichem Wasser und gegebenenfalls dem Dispersionsmedium, das das Polysaccharid nicht löst, vorlegt, je nach Art des Polysaccharids gegebenenfalls eine Vorbehandlung anschließt und dann den Kohlensäureester zudosiert.

Eine Vorbehandlung erfolgt zweckmäßig dann, wenn das Polysaccharid, wie im Falle der Cellulose für die Umsetzung aufgeschlossen werden soll. Die Temperatur kann zwischen 10 und 120°C variieren und ist nicht kritisch. Die Zeit für die Vorbehandlung ist ebenfalls nicht kritisch. Sie kann zwischen mehreren Minuten und vielen Stunden liegen. In der Regel liegt sie zwischen 1 bis 30 h. Eine solche Vorbehandlung ist jedoch nicht zwingend vorgeschrieben, da der notwendige Aufschluß auch während der Umsetzung erfolgen kann.

Man kann auch mit wäßrigem Alkalihydroxid vorbehandelte, anschließend von Alkalihydroxid und überschüssigem Wasser befreite Polysaccharide in die Reaktion einsetzn. Dies kann besonders bei Cellulosen in einzelnen Fällen vorteilhaft sein und aufgrund eines besseren Aufschlusses den Substitutionsgrad noch etwas erhöhen. Dafür eignen sich besondere 8 bis 25 gew.-%ige Natronlaugen, die anschließend mit Wasser ausgewaschen werden und das Wasser mit Alkoholen wie Isopropanol oder der Hilfsbase. Der Alkohol kann durch Trocknen oder Verdrängen mit dem Dispersionsmedium für die Umsetzung entfernt werden.

Der Kohlensäureester wird mit einer solchen Geschwindigkeit zudosiert, daß die dabei freiwerdende Wärme kontrolliert abgeführt werden kann.

Die Reaktionstemperatur beträgt im allgemeinen zwischen 10 und 120°C, vorzugsweise 15° und 100°C, besonders bevorzugt 20° und 90°C. Je empfindlicher das Polysaccharid ist, desto niedriger wird man die Temperatur wählen.

Die Reaktionsdauer hängt von der Art des Polysaccharids ab und von der gewählten Temperatur. Je besser die Polysaccharide aufgeschlossen sind, desto kürzer kann die Reaktionszeit sein. Um einen ausreichenden DS zu erzielen, sollte sie jedoch 3 h nicht unterschreiten. Längere Reaktionszeiten sind im Gegensatz zu den o.a. diskutierten Literaturangaben jedoch nicht schädlich und führen in der Regel zu einem weiteren Anstieg des DS. Für eine möglichst vollständige Nutzung des Acylierungsreagenzes empfiehlt sich also eine verlängerte Reaktionszeit, die natürlich zusammen mit anderen ökonomischen Fakten und einer gegebenenfalls möglichen Schädigung eines empfindlichen Polysaccharids betrachtet werden muß.

Die Aufarbeitung der Polysaccharidcarbonate erfolgt nach an sich bekannten Methoden und richtet sich natürlich nach der Natur des umgesetzten Polysaccharids. Sie gestaltet sich besonders einfach, wenn man nach Abtrennen des flüssigen Teils des Reaktionsgemisches das Reaktionsprodukt durch Waschen mit geeigneten inerten Lösungsmitteln von den anhaftenden Resten des Reaktionsgemisches befreien kann. Dabei sollte weder Lösung noch Quellung erfolgen. Die geeigneten Lösungsmittel müssen von Fall zu Fall durch Vorversuche ermittelt werden. Vielfach sind die o.a. Lösungsmittel brauchbar. Häufig können auch Alkohole oder Wasser verwendet werden.

Die nach dem erfindungsgemäßen Verfahren hergestellten erfindungsgemäßen Polysaccharidcarbonate besitzen Substitutionsgrade von 0,5 bis 3,0, bevorzugt von 0,8 bis 3,0, besonders bevorzugt von 1,0 bis 3,0. Selbstverständlich können nach dem erfindungsgemäßen Verfahren auch Polysaccharidcarbonate mit geringeren Substitutionsgraden von kleiner 0,5 bis 0,1 hergestellt werden, falls dies gewünscht wird.

Die erfindungsgemäßen Polysaccharidcarbonate, besonders die Phenylcarbonate, sind hervorragende Ausgangsprodukte zur Herstellung verschiedener weiterer Derivate, besonders zur Herstellung von Carbamaten und zur Fixierung von katalytisch aktiven Spezies, z.B. von Enzymen. Sie können auch durch Umsetzung mit Polyaminen in basische Polysaccharide umgewandelt werden, die als basische Ionenaustauscher geeignet sind. Derartige basische Polysaccharide können auch durch Umsetzung mit Alkylierungsmittel in quartäre Ammoniumgruppen enthaltende Polysaccharide übergeführt werden.

Durch die hohen Substitutionsgrade der Carbonate lassen sich entsprechend hohe Substitutionsgrade bei der Carbamatbildung und der Kationisierung und damit höhere Effizienz bei der Anwendung erreichen. Kationische Polysaccharide jedoch sind begehrte Filterhilfsmittel für medizinische Filter, Hilfsmittel für die Papierherstellung, Zusätze zu kosmetischen Reinigungsmitteln, Flockungsmittel für die Abwasserklärung (siehe auch M. Langer, Wochenblatt für Papierfabrikation 1978 (18), 690 bis 693).

Je nach eingesetztem Polysaccharid können auf dem o.a. Wege sowohl unlösliche, feste wie auch lösliche, basische oder kationische Polysaccharide hergestellt werden.

Geht man beispielsweise von hochmolekularen Cellulosen aus, so gewinnt man unlösliche Produkte mit faseriger Struktur, die hervorragend geeignet sind zur Herstellung von Filtermaterialien oder zur Enzymfixierung.

Aus Stärke beispielsweise sind lösliche Produkte zugänglich. Ähnliches gilt für Polysaccharide mit kleinerem Molgewicht wie Dextrine. Auch aus Celluloseethern können lösliche, basische und kationische Produkte erhalten werden. Geeignet sind dafür beispielsweise Methylcellulose, Ethylcellulose und Carboxymethylcellulose.

Solche löslichen Produkte können vorteilhaft für die Papierherstellung, als Flockungsmittel und für Reinigungsmittel eingesetzt werden.

Die erfindungsgemäßen Polysaccharidcarbonate sind hervorragend geeignet für weitere Umsetzungen, vor allem für die Herstellung von Carbamaten sowie basische Polysaccharidurethanamine. Insbesondere sind die so hergestellten basischen Polysaccharide hervorragend geeignet für die Herstellung kationischer Polysaccharide.

### Beispiele

### Anmerkungen:

Die Molangaben zu den Mengen an Polysaccharid beziehen sich auf die Monomereinheit. Auch wenn es nicht besonders erwähnt ist, werden alle Reaktionen an suspendiertem, nicht gelöstem Polysaccharid durchgeführt. Elementaranalysen werden in der Regel an bei 105°C im Vakuum getrockneten Proben vorgenommen.

### Beispiel 1

Eine Suspension von 200 g (∼1,25 Mol) einer lufttrockenen handelsüblichen Fichtencellulose mit einem Feuchtegehalt von 6 bis 7 Gew.-% in 900 g über KOH getrocknetem Pyridin und 2,5 l über CaCl₂ getrocknetem Benzol wurde 20 h bei Raumtemperatur gerührt, über 3 h tropfenweise bei Raumtemperatur mit 785 g (5,0 Mol) Phenylchlorkohlensäureester versetzt, 70 h bei Raumtemperatur und dann noch 6 h bei 80°C gerührt. Nach Abkühlen und Absaugen wurde 1 x mit Benzol, 2 x mit Isopropanol und 3 x mit Wasser gründlich gewaschen und 3 h bei 105°C im Vakuum getrocknet. Ausbeute: 656 g eines trockenen Pulvers

| | | | | | | |
|---|---|---|---|---|---|---|
| Elementaranalyse: | C | 60,7 % | H | 4,3 % | O | 32,6 % |

Der DS liegt um 3.

### Beispiel 2

106 g (∼0,5 Mol) einer Hydroxyethylcellulose mit einem Wassergehalt von 6,5 Gew.-% und einem DS von 1,1 werden in 1 l Benzol und 198 g (2,5 Mol) Pyridin (beide getrocknet) suspendiert und nach 20 h Rühren bei 50°C im Verlaufe von 2 h mit 392 g (2,5 Mol) Chlorkohlensäurephenylester versetzt. Nach weiteren 20 h Rühren bei 80°C wird abgekühlt, abgesaugt mit Benzol und Isopropanol salzfrei gewaschen und im Hochvakuum bei 50°C bis zur Gewichtskonstanz getrocknet.
Ausbeute: 260 g mit einem DS von 2,4.

| | | | | |
|---|---|---|---|---|
| Elementaranalyse: | C | 59,4 % | H | 4,7 % |
| (Ausgangsprodukt: | | 46,2 % | | 7,5 %) |

IR-Spektrum starke Carbonat-CO-Bande bei 1770 cm⁻¹

### Beispiel 3

Eine Malte aus langfaseriger handelsüblicher Cellulose wird zerpflückt über Nacht in Wasser gequollen, die Flocken im Starmix zerkleinert, abgesaugt und gepreßt, ca. 40 h bei 105°C im Vakuum bis zur Gewichtskonstanz getrocknet und noch vorhandene Flocken im Starmix zerfasert.

81 g (0,5 Mol) der so vorbereiteten Cellulose werden in 1,5 l Methylenchlorid und 158 g (2,0 Mol) Pyridin (beide über Baylith T 144*) getrocknet) nach Zugabe von 2,5 g (0,14 Mol) Wasser suspendiert und 20 h bei Raumtemperatur vorbehandelt. Bei 20 bis 25°C werden in 3 h 235,5 g (1,5 Mol) Chlorkohlensäurephenylester zugetropft und das Gemisch wird noch 20 h bei 25°C und 5 h bei 45°C gerührt. Nach Abkühlen, Abdrücken, Waschen mit Methylenchlorid und Isopropanol wird der Filterrückstand bei 50°C im Vakuum bis zur Gewichtskonstanz getrocknet.
Ausbeute: 141 g faseriges Material
^{*)} Zeolith A der Bayer AG

| | | | | |
|---|---|---|---|---|
| Elementaranalyse: | C | 55,0 % | H | 5,1 % |

DS 1,0 bis 1,2
Wiederholt man diesen Versuch mit der hochgetrockneten Cellulose ohne Wasserzusatz, so findet keine Umsetzung statt.

### Beispiel 4

Zu einer Suspension von 183 g (1 Mol) Methylcellulose (4 bis 5 % H₂O) mit einem Substitutionsgrad von 1,4 in 316 g (4,0 Mol) Pyridin und 2,0 l Methylenchlorid (beide getrocknet mit Molekularsieb Baylith T 144), die 15 h bei Raumtemperatur gerührt wurde, tropft man in 3 h 314 g (2,0 Mol) Chlorkohlensäurephenylester bei Raumtemperatur hinzu, rührt noch 20 h bei 20 bis 25°C, saugt ab, wäscht mit Methylenchlorid und Isopropanol salzfrei und trocknet im Vakuum bei 50°C bis zur Gewichtskonstanz. Man erhält 316 g eines lockeren Materials mit einem DS von 1,3.

| | | | | |
|---|---|---|---|---|
| Elementaranalyse: | C | 58,6 % | H | 5,29 % |
| (Methylcellulose: | | 49,2 % | | 7,1 %) |

### Beispiel 5

Zu einer Suspension von 46 g (0,25 Mol) Methylcellulose (DS 1,4; ca, 5 % H₂O) in 800 ml Benzol und 79 g Pyridin (1,0 Mol), die 15 h bei 25°C gerührt worden war, tropft man in 3 h 109 g (1,0 Mol) Chlorkohlensäureethylester bei RT zu und hält noch 5 h bei 22 bis 25°C. Man saugt ab, wäscht mit Benzol und Isopropanol chloridfrei und trocknet im Vakuum bei 50°C bis zur Gewichtskonstanz.
Ausbeute: 67 g mit einem DS von 1,0

| | | | | |
|---|---|---|---|---|
| Elementaranalyse: | C | 48,6 % | H | 6,6 % |

IR-Spektrum: starke Carbonat-CO-Bande bi 1770 cm⁻¹

### Beispiel 6

Zu einer Suspension von 183 g (1,0 Mol) Methylcellulose (ca. 5 % H₂O, DS 1,4), 316 g (4,0 Mol) Pyridin und 1,5 l Methylenchlorid (beide mit Zeolith A getrocknet) tropft man in 3 h 47 g (0,30 Mol) Chlorkohlensäurephenylester und rührt 20 h bei 25°C und 5 h bei 45°C. Nach Aufarbeitung mit Methylenchlorid und Isopropanol und Trocknen bei 50°C erhält man 210 g.

### Beispiel 7

162 g (1,0 Mol) Fichtencellulose (6 % H₂O) werden 3 h mit 18 %iger NaOH behandelt, abgesaugt, mit Wasser alkalifrei gewaschen und mit Pyridin wasserfrei. Zu der Suspension in Pyridin gibt man in 2 h 314 g (2 Mol) Chlorameisensäurephenylester und hält 12 h bei 80°C. Man saugt ab, wäscht mit Isopropanol oder Wasser und trocknet bei 50°C im Vakuum.
Ausbeute: 439 g, DS ca. 2.

### Beispiel 8

53 g (0,25 Mol) Hydroxyethylcellulose (6-7 % H₂O, DS 1,1), 100 g Pyridin und 100 g Chlorkohlensäurephenylester werden in Benzol 24 h bei 20°C und 5 h bei 80°C gerührt. Man wäscht nach Absaugen mit Isopropanol und trocknet im Vakuum bei 50°C.
Ausbeute: 100 g mit einem DS von 1,3-1,4.

| | | | | |
|---|---|---|---|---|
| Elementaranalyse: | C | 55,3 % | H | 6,5 %. |

### Beispiel 9

100 g (0,625 Mol) Stärke, 24 h bei Raumtemperatur in 197,5 g (2,5 Mol) Pyridin und 1 l Benzol gerührt, werden in 4 h tropfenweise mit 290 g (1,85 Mol) Chlorkohlensäurephenylester versetzt und noch 10 h bei Raumtemperatur und 20 h bei 80°C gerührt. Absaugen, waschen mit Isopropanol/Wasser (1:2) und Trocknen im Hochvakuum ergibt einen schwach rosa gefärbten Feststoff.
Ausbeute: 200 g, DS 1,5

| | | | | |
|---|---|---|---|---|
| Elementaranalyse: | C | 58,9 % | H | 4,5 % |

IR-Spektrum: starke Carbonat-CO-Bande

### Beispiel 10

44 g (0,25 Mol) einer Methylcellulose mit ca. 5 % Wasser und einem DS von 1,0, 79 g (1,0 Mol) Pyridin und 600 ml Chloroform werden 15 h bei 65°C gerührt, in 3 h bei 10°C tropfenweise mit 157 g (1,0 Mol) Chlorkohlensäurephenylester versetzt, 13 h bei 50 bos 60°C gerührt, abgesaugt, chloridfrei gewaschen und getrocknet.
Ausbeute: 106 g mit einem DS von 2,0

| | | | | |
|---|---|---|---|---|
| Elementaranalyse: | C | 60,2 % | H | 4,3 % |

## Patentansprüche

1. Polysaccharidcarbonate mit wiederkehrenden Einheiten der allgemeinen Formel 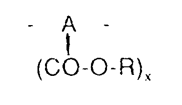 worin bedeuten
A eine Cellulose-, Methylcellulose-, Ethylcellulose-, Hydroxyethylcellulose-, Hydroxypropylcellulose-, Carboxymethylcellulose-, Na-, K-, Ca-, NH₄-, quartäre Ammoniumsalze der Carboxymethylcellulose-, Na-, K-, Ca-, NH₄-, quart. Ammonium-Cellulosesulfat-, Stärke-, Dextrin-, Glycogen-, Fructose-, Inulin-, Graminin-, Mannose-, Galactosan-, Hemicellulose-, Xylose-, Arabinose-, Gellan-, Xanthan- oder Pullulan-Einheit
R einen aliphatischen oder cycloaliphatischen Rest mit 1 bis 18 C-Atomen, einen araliphatischen Rest mit 7 bis 12 C-Atomen, einen gegebenenfalls mit Halogen, NO₂, Phenyl, COOR¹, OR¹ oder einen mit einem C₁-C₆-Aliphaten substituierten aromatischen Rest mit 6 bis 12 C-Atomen
R¹ Alkyl mit 1 bis 4 C-Atomen
x eine Zahl von 0,5 bis 3,0
wobei die Cellulose- bzw. Cellulosederivat- bzw. Stärkecarbonate bei 20°C zu maximal 20 Gew.-% in Chloroform löslich sind und von den Carbonatgruppen maximal 30 % cyclische Carbonatgruppen sind.

2. Cellulose- bzw. Cellulosederivat- bzw. Stärkecarbonate gemäß Anspruch 1. dadurch gekennzeichnet, daß bedeuten:
R einen aliphatischen Rest mit 1 bis 6 C-Atomen, einen aromatischen Rest mit 6 bis 10 C-Atomen gegebenenfalls substituiert mit Cl, CH₃, NO₂, OCH₃ und COOCH₃ und
x eine Zahl von 0,8 bis 3,0

3. Cellulose- bzw. Cellulosederivat- bzw. Stärkecarbonate gemäß Anspruch 1, dadurch gekennzeichnet, daß bedeuten:
R CH₃, C₂H₅, Phenyl, Chlorphenyl, Nitrophenyl und Naphthyl und
x eine Zahl von 1,0 bis 3,0.

4. Polysaccharidcarbonate gemäß Anspruch 1, dadurch gekennzeichnet, daß bedeuten:
R Phenyl und
x eine Zahl von 1,0 bis 3,0.

5. Verfahren zur Herstellung von Cellulose- bzw. Cellulosederivat- bzw. Stärkecarbonaten gemäß einem der Ansprüche 1-4 durch Umsetzung von Cellulose- bzw. Cellulosederivaten bzw. Stärke mit Kohlensäureestern in Gegenwart einer Base, dadurch gekennzeichnet, daß man die Cellulose- bzw. Cellulosederivaten in heterogener Phase, gegebenenfalls nach Vorbehandlung mit der Base, in Gegenwart von mindestens 0,5 Gew.-% Wasser mit wenigstens einem Kohlensäureester bei Temperaturen von 10 - 120°C und in einem Molverhältnis Base zu Kohlensäureester zwischen 1:1 bis 10:1.

## Claims

1. Polysaccharide carbonates containing recurring units corresponding to the following general formula: 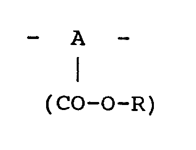 in which
A is a cellulose, methyl cellulose, ethyl cellulose, hydroxyethyl cellulose, hydroxypropyl cellulose, carboxymethyl cellulose, Na, K, Ca, NH₄, quaternary ammonium salts of carboxymethyl cellulose, Na, K, Ca, NH₄, quat. ammonium cellulose sulfate, starch, dextrin, glycogen, fructose, inulin, graminin, mannose, galactose, hemicellulose, xylose, arabinose, gellan, xanthan or pullulan unit,
R is an aliphatic or cycloaliphatic containing 1 to 18 carbon atoms, an araliphatic radical containing 7 to 12 carbon atoms, an aromatic radical containing 6 to 12 carbon atoms optionally substituted by halogen, NO₂, phenyl, COOR¹, OR¹ or by a C₁₋₆ aliphatic group,
R¹ is a C₁₋₄ alkyl group,
x is a number of 0.5 to 3.0,
at most 20% by weight of the cellulose or cellulose derivative or starch carbonates being soluble in chloroform at 20°C and at most 30% of the carbonate groups being cyclic carbonate groups.

2. Cellulose or cellulose derivative or starch carbonates as claimed in claim 1, characterized in that
R is an aliphatic radical containing 1 to 6 carbon atoms, an aromatic radical containing 6 to 10 carbon atoms optionally substituted by Cl, CH₃, NO₂, OCH₃ and COOCH₃ and
x is a number of 0.8 to 3.0.

3. Cellulose or cellulose derivative or starch carbonates as claimed in claim 1, characterized in that
R is CH₃, C₂H₅, phenyl, chlorophenyl, nitrophenyl and naphthyl and
x is a number of 1.0 to 3.0.

4. Polysaccharide carbonates as claimed in claim 1, characterized in that
R is phenyl and
x is a number of 1.0 to 3.0.

5. A process for the production of the cellulose or cellulose derivative or starch carbonates claimed in any of claims 1 to 4 by reaction of cellulose or cellulose derivatives or starch with carbonic acid esters in the presence of a base, characterized in that, optionally after pretreatment with the base, the cellulose or cellulose derivatives are reacted in heterogeneous phase with at least one carbonic acid ester in the presence of at least 0.5% by weight of water at temperatures of 10 to 120°C and in a molar ratio of base to carbonic acid ester of 1:1 to 10:1.

## Revendications

1. Carbonates de polysaccharides à motifs récurrents de formule générale 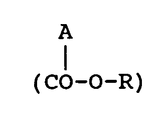 dans laquelle
A représente un motif cellulose, méthylcellulose, éthylcellulose, hydroxyéthylcellulose, hydroxypropylcellulose, carboxyméthylcellulose, sels de Na, K, Ca, NH₄, ammonium quaternaire de carboxyméthylcellulose, cellulose-sulfate de Na, K, Ca, NH₄ ou ammonium quaternaire, amidon, dextrine, glycogène, fructose, inuline, graminine, mannose, galactosane, hémicellulose, xylose, arabinose, gellane, xanthane ou pullulane
R est un reste aliphatique ou cycloaliphatique ayant 1 à 18 atomes de carbone, un reste araliphatique ayant 7 à 12 atomes de carbone, un reste aromatique de 6 à 12 atomes de carbone éventuellement substitué avec un halogène, un radical NO₂, phényle, COOR¹, OR¹ ou avec un radical aliphatique en C₁ à C₆,
R¹ est un groupe alkyle de 1 à 4 atomes de carbone
x est un nombre de 0,5 à 3,0,
les carbonates de cellulose ou de dérivés de cellulose ou d'amidon étant solubles à 20°C en proportion maximale de 20 % en poids dans le chloroforme et un maximum de 30 % des groupes carbonate étant des groupes carbonate cycliques.

2. Carbonates de cellulose ou de dérivés de cellulose ou d'amidon suivant la revendication 1, caractérisés en ce que :
R est un reste aliphatique ayant 1 à 6 atomes de carbone, un reste aromatique ayant 6 à 10 atomes de carbone éventuellement substitué par Cl, CH₃, NO₂, OCH₃ et COOCH₃ et
x est un nombre de 0,8 à 3,0.

3. Carbonates de cellulose ou de dérivés de cellulose ou d'amidon suivant la revendication 1, caractérisés en ce que :
R est choisi entre les groupes CH₃, C₂H₅, phényle, chlorophényle, nitrophényle et naphtyle et
x est un nombre de 1,0 à 3,0.

4. Carbonates de polysaccharides suivant la revendication 1, caractérisés en ce que :
R est un groupde phényle et
x est un nombre de 1,0 à 3,0.

5. Procédé de production de carbonates de cellulose ou de dérivés de cellulose ou d'amidon suivant l'une des revendications 1 à 4 par réaction de cellulose ou de dérivés de cellulose ou d'amidon avec des esters d'acide carbonique en présence d'une base, caractérisé en ce qu'on fait réagir la cellulose ou les dérivés de cellulose en phase hétérogène, éventuellement après traitement préalable avec la base, en présence d'au moins 0,5 % en poids d'eau, avec au moins un ester d'acide carbonique à des températures de 10 à 120°C et dans un rapport molaire de la base à l'ester d'acide carbonique compris entre 1:1 et 10:1.
